## Europäisches Patentamt
## European Patent Office
## Office européen des brevets

(19)

(11) Veröffentlichungsnummer: **0 190 766**
**B1**

(12) # EUROPÄISCHE PATENTSCHRIFT

(45) Veröffentlichungstag der Patentschrift:
31.05.89

(21) Anmeldenummer: 86101608.7

(22) Anmeldetag: 07.02.86

(51) Int. Cl.⁴: **C 12 Q 1/38** //
**C12Q1/56**

(54) **Bestimmung von Prokallikrein.**

(30) Priorität: 08.02.85 DE 3504405

(43) Veröffentlichungstag der Anmeldung:
13.08.86 Patentblatt 86/33

(45) Bekanntmachung des Hinweises auf die Patenterteilung:
31.05.89 Patentblatt 89/22

(84) Benannte Vertragsstaaten:
AT BE CH DE FR GB IT LI LU NL SE

(56) Entgegenhaltungen:
US-A-4 016 042

CLINICAL CHEMISTRY, Band 27, Nr. 4, April 1981,
Seiten 586-593, Easton, Pennsylvania, US; R. ITO et
al.: "Centrifugal analysis for plasma kallikrein
activity, with use of the chromogenic substrate
S-2302"
H.U. BERGMEYER "Methods of Enzymatic
Analysis", Band V "Enzymes 3: Peptidases,
Proteinases and Their Inhibitors", 3. Auflage, 1983,
Seiten 411-414, Verlag Chemie, Weinheim;
CLINICAL CHEMISTRY, Band 29, Nr. 2, Februar
1983, Seiten 225-236, Washington, US; J. FAREED et
al.: "Diagnostic efficacy of newer syntheticsubstrates methods for assessing coagulation
variables: A critical overview"
CHEMICAL ABSTRACTS, Band 93, Nr. 23, 8.
Dezember 1980, Seite 202, Spalte 1,

(73) Patentinhaber: BOEHRINGER MANNHEIM GMBH,
Patentabteilung, Abt. E Sandhofer Strasse 112-
132 Postfach 31 01 20, D-6800 Mannheim 31
Waldhof (DE)

(72) Erfinder: Bartl, Knut, Dr. rer. nat., Am Westend 6,
D-8121 Wilzhofen (DE)
Erfinder: Lill, Helmut, Dr. rer. nat.,
Zugspitzstrasse 24, D-8121 Wielenbach (DE)

(74) Vertreter: Weickmann, Heinrich, Dipl.- Ing.,
Patentanwälte Dipl.- Ing. H.Weickmann Dipl.-
Phys.Dr. K.Fincke Dipl.- Ing. F.A.Weickmann
Dipl.- Chem. B. Huber Dr.- Ing. H. Liska Dipl.-
Phys.Dr. J. Prechtel Postfach 860620, D-8000
München 86 (DE)

(56) Entgegenhaltungen: (Fortsetzung)
Zusammenfassungsnr. 217028v, Columbus, Ohio,
US; S. NAKAMURA et al.: "Determination of
plasma prekalikrein with chromogenic peptide
substrate", & RINSHO BYORI 1980, 28(7), 670-674
CHEMICAL ABSTRACTS, Band 92, Nr. 19, 12. Mai
1980, Seite 205, Spalte 1, Zusammenfassungsnr.
159405g, Columbus, Ohio, US; P. FRIBERGER et al.:
"Determination of prekallikrein in plasma by
means of a chromogenic tripeptide substrate for
plasma kallikrein", & EXP. MED. BIOL. 1978
(veröffentlicht 1979) 67-82

## Beschreibung

Die Erfindung betrifft ein Verfahren zur simultanen Bestimmung von Prokallikrein und der partiellen Thromboplastinzeit (PTT) im Plasma. Aus der Bestimmung von Prokallikrein können Aussagen über das Blutgerinnungssystem getroffen werden. Das Fehlen von Prokallikrein äußert sich in einer verlängerten Gerinnungszeit. Bei verlängerten Gerinnungszeiten kann die Prokallikreinbestimmung Aufschluß darüber geben, ob diese Verlängerung durch einen Mangel an Faktor VIII, IX oder durch einen Prokallikreinmangel verursacht wird. Ergibt die Prokallikreinbestimmung, daß es sich um einen Mangel an Faktor VIII oder IX handelt, so deutet dies auf ernsthafte Erkrankungen hin. Außerdem wird auch bei bestimmten anderen Krankheitszuständen wie z. B. Schock, eine Veränderung der Prokallikreinmenge festgestellt.

Die Kallikreinbildung aus Prokallikrein im Plasma läuft nach folgendem Reaktionsschema ab:

$$\text{Faktor XII} \xrightarrow{\quad\text{Dextransulfat}\quad} \text{Faktor XIIa}$$

$$\text{Prokallikrein} \xrightarrow{\quad\text{Faktor XIIa}\quad} \text{Kallikrein}$$

Die bekannten Methoden zur Bestimmung von Prokallikrein sind in H.U. Bergmeyer, Methods of Enzymatic Analysis, 3. Auflage, Band V, Seite 412/413 beschrieben. Neben einigen sehr komplizierten und zeitaufwendigen Methoden, die für eine Routinebestimmung im Gerinnungslabor ausscheiden, ist es danach bekannt, nach Aktivierung von Faktor XII mit Dextransulfat gebildetes Kallikrein über ein chromogenes Substrat zu bestimmen. Dieses Verfahren hat jedoch den Nachteil, daß die Inkubation zur Überführung von Prokallikrein in Kallikrein bei 0°C durchgeführt werden muß, um den Einfluß von Plasmainhibitoren wie z. B. alpha$_1$-Antitrypsin und C$_1$-Inaktivator auf das Meßergebnis zu verhindern. Die Inkubation bei 0°C wiederum behindert die Automatisierbarkeit dieses Verfahrens schwerwiegend.

Der Erfindung liegt die Aufgabe zugrunde, ein einfaches und schnelles Verfahren zur Bestimmung von Prokallikrein und simultan der partiellen Thromboplastinzeit zu schaffen, welches die erwähnten Nachteile nicht aufweist und insbesondere bei Normaltemperatur durchgeführt werden kann.

Gelöst wird diese Aufgabe gemäß der Erfindung durch ein Verfahren zur simultanen photometrischen Bestimmung von Prokallikrein und der partiellen Thromboplastinzeit (PTT) im Plasma, das dadurch gekennzeichnet ist, daß man Plasma mit einem Oberflächenaktivator aus der Gruppe Ellagsäure, Sulfatide und feinteiliges Siliciumdioxid inkubiert, dann ein chromogenes Thrombinsubstrat zusetzt, die erhaltene Extinktion mißt und danach die daraus freigesetzte optisch bestimmbare Gruppe in kurzen Zeitabständen oder kontinuierlich mißt und den Anstieg des linearen Teils der beim Auftrag der Zeit gegen die Extinktion erhaltenen Kurve als Maß für den Prokallikreingehalt und die Zeit bis zum Erreichen einer vorbestimmten Extinktion als PTT bestimmt.

Der Oberflächenaktivator wird unter Ellagsäure, Sulfatiden (vorzugsweise aus Hirnmaterial) und feinteiligem Siliciumdioxid, wie es z. B. unter der Bezeichnung Aerosil®, Degussa, Deutschland, im Handel ist, ausgewählt. Dextransulfat ist nicht geeignet. Besonders bevorzugt wird Ellagsäure in einer Konzentration von 0,08 bis 2,1 µg/ml Testlösung. Die besten Ergebnisse werden mit einer Konzentration von 0,40 bis 0,45 µg Ellagsäure pro ml erhalten. Diese und alle folgenden Konzentrationsangaben in dieser Beschreibung beziehen sich auf die Endkonzentrationen im Test.

Überraschenderweise ist es beim Verfahren der Erfindung möglich, bei normalen Temperaturen, insbesondere bei 20 bis 40°C und vorzugsweise bei 30 bis 37°C, zu inkubieren.

Als chromogene Thrombinsubstrate können die hierfür handelsüblichen Peptide verwendet werden, welche eine chromogene Gruppe, insbesondere eine Paranitroanilingruppe oder ein Derivat derselben, enthalten, welche unter Enzymeinwirkung aus dem Substrat abgespalten wird und dann optisch bestimmt werden kann, soweit sie für die PTT-Bestimmung genügend empfindlich sind. Bevorzugte chromogene Thrombinsubstrate für die Verwendung im Rahmen der Erfindung sind Tos-Gly-Pro-Arg-pNA, H-D-CHA-Ala-Arg-pNA, H-D-CHG-Gly-Arg-pNA, H-D-CHG-Ala-Arg-pNA, H-D-CHG-but-Arg-pNA, Iso-Buco-Gly-Arg-pNA, Naphthyl-SO$_2$-Gly-Pro-Arg-pNA, H-D-Phe-Pip-Arg-pNA und Bz-Pro-Phe-Arg-pNA.

Die Konzentration des chromogenen Thrombinsubstrats beträgt zweckmäßig 0,01 bis 2 mMol/l, vorzugsweise etwa 0,02 bis 0,04 mMol/l in Abhängigkeit von der Michaelis-Konstante des jeweiligen Substrats. Besonders bevorzugt wird als Substrat Tos-Gly-Pro-Arg-p-Nitroanilin und Bz-Pro-Phe-Arg-pNA.

Vorzugsweise wird das Verfahren der Erfindung in Gegenwart von tierischen, pflanzlichen oder synthetischen Phospholipiden durchgeführt. Beispiele für geeignete Phospholipide sind Kephalin, Sojabohnen-Phospholipid, Phosphatidylethanolamin und Phosphatidylcholin. Zahlreiche andere Phospholipide sind jedoch ebenfalls geeignet, können aufgrund ihrer großen Zahl hier aber nicht speziell aufgeführt werden. Besonders bevorzugt wird Kephalin. Seine Menge liegt zweckmäßig zwischen 4 und 200 µg/ml. Im Falle einer Simultanbestimmung von PTT ist ein Zusatz eines Phospholipids notwendig.

Desweiteren ist es zweckmäßig, das erfindungsgemäße Verfahren in Gegenwart von Calciumionen durchzuführen, da Calciumzusatz eine Empfindlichkeitssteigerung des Verfahrens bewirkt durch Erhöhung der Extinktionsdifferenz pro Zeiteinheit. Bevorzugt wird eine Konzentration von 25 bis 40 µMol/ml Ca$^{2+}$-Ionen. Im

Falle der Simultanbestimmung von PTT ist der Calciumionenzusatz erforderlich. Bei Zusatz von $Ca^{2+}$-Ionen und Phospholipid ergibt sich eine S-förmige Kurve, deren Grundlinie für die Prokallikreinbestimmung verwendet wird.

Die Inkubationszeit liegt beim Verfahren der Erfindung zwischen 1 und 10 Minuten, wobei 4 bis 6 Minuten bevorzugt werden. Bei zu langer oder zu kurzer Inkubationszeit erhält man niedrigere Prokallikreinwerte.

Als Plasma wird im Rahmen der Erfindung z. B. Citratplasma, Oxalatplasma oder EDTA-Plasma verwendet.

Wird das Verfahren der Erfindung in Gegenwart von Calciumionen durchgeführt, so erfolgt die Messung in den ersten knapp 30 Sekunden nach Zugabe des Plasmas, da anschließend die Extinktion steil ansteigt. Ohne Calciumionenzusatz erfolgt dieser steile Anstieg nicht und die Messung kann auch in längeren Zeitabständen durchgeführt werden.

Die Bildung des Chromophors aus dem chromogenen Substrat wird photometrisch verfolgt. Dies kann sowohl kinetisch durch Bestimmung der Extinktionsänderungen pro Zeiteinheit oder durch Zweipunktkinetik als auch durch Endpunktmessung erfolgen. Im Falle einer Endpunktmessung muß die Farbbildungsreaktion abgestoppt werden, was bevorzugt durch Säurezusatz erfolgt. Besonders geeignet zum Abstoppen sind beispielsweise Essigsäure oder Zitronensäure. Im Falle der Essigsäure wird diese bevorzugt in einer Konzentration von 20 bis 100 Vol.-%, im Falle von Zitronensäure in einer Konzentration von 5 bis 20 Vol.-% angewendet.

Die folgenden Beispiele erläutern die Erfindung weiter in Verbindung mit der beigefügten Zeichnung. In dieser stellen dar:

Fig. 1    eine graphische Darstellung der Extinktionsdifferenz pro Zeiteinheit bei unterschiedlichem Prokallikreingehalt im Plasma.

Fig. 2    eine graphische Darstellung der Extinktion gegen die Reaktionszeit aufgetragen, aus welcher man den S-förmigen Kurvenverlauf erkennen kann.

Fig. 3    eine graphische Darstellung, welche die Korrelation der PTT-Bestimmung im Simultantest mit der Erfindung mit einem bekannten PTT-Testverfahren zeigt.

## Beispiel

Simultanbestimmung von Prokallikrein und PTT

**Reagenz 1:**

| | |
|---|---|
| Puffer (Tris/HCl) | 100 μmol/ml, pH 7,6 |
| Ellagsäure | 0,5 μg/ml |
| Kephalin aus Kaninchenhirn | 0,04 mg/ml |
| Merthiolat | 0,01 Gew.-% |

**Reagenz 2:**

| | |
|---|---|
| Tos-Gly-Pro-Arg-p-NA | 0,376 μmol/ml |
| $Ca^{2+}$-Ionen | 40 μml/ml |

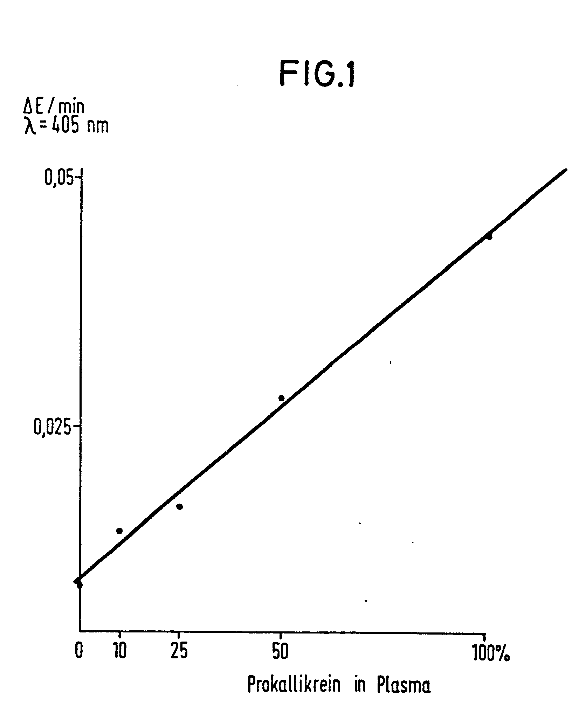

Zu 2,0 ml Reagenz 1 werden 0,2 ml Probe (Citratplasma) gegeben und bei 37°C 5 Minuten inkubiert. Anschließend wird 0,2 ml Reagenz 2 zugegeben, gemischt und sofort die photometrische Bestimmung bei 37°C und 405 nm durchgeführt, wobei zur Bestimmung von Prokallikrein ΔE/min innerhalb 25 Sekunden bestimmt wird. Danach läßt man die Reaktion weiter laufen und führt eine "fixed absorbance-Messung" durch. Hinrbei wird die Zeit bis zum Erreichen einer bestimmten Extinkitonsdifferenz zum Leerwert (ΔE = 0,2) gemessen. Diese Zeit ist die PTT-Zeit. Bei verschiedenen Prokallikreingehalten im Plasma erhält man als Ergebnis Meßwerte gemäß Fig. 1. Dabei bedeutet 100 % Prokallikrein im Plasma den Prokallikreingehalt eines Normalplasmapools.

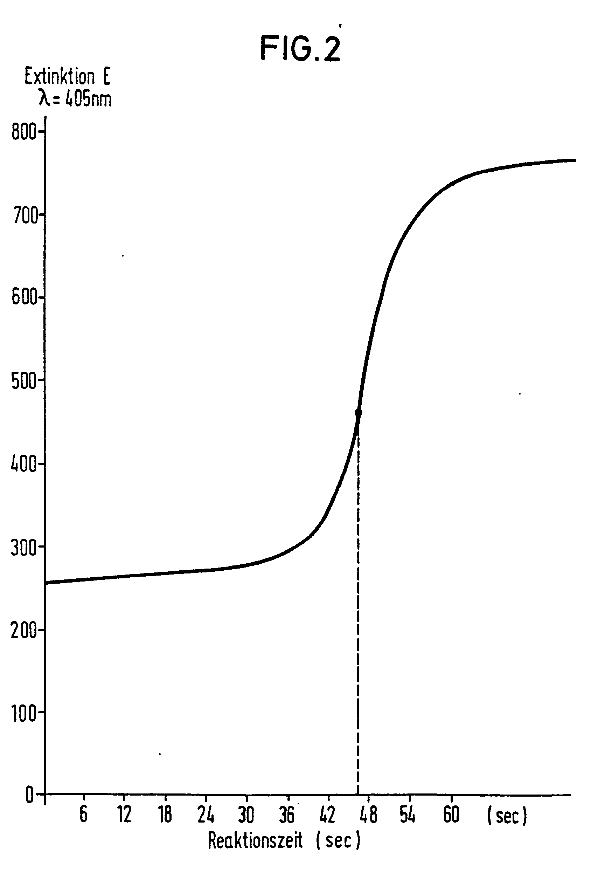

Figur 2 zeigt den mit einem Schreiber aufgezeichneten Extinktionsverlauf. (PTT-Zeit: 46,3 Sekunden; Prokallikrein-Reaktion: 0,042 ΔE/min).

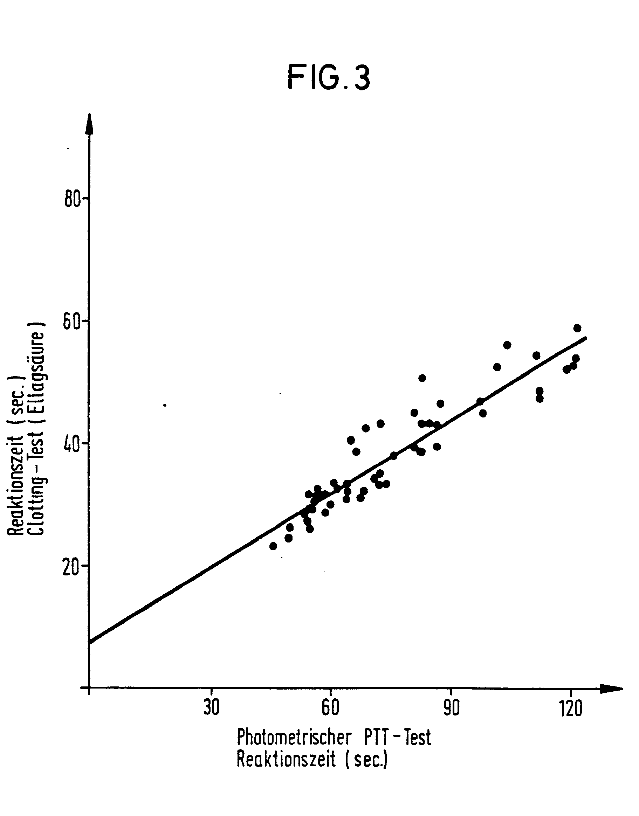

Figur 3 zeigt die Korrelation der PTT-Bestimmung gemäß diesem Beispiel mit einem herkömmlichen PTT-Test. (Clotting-Methode, Actin-Test[7], Merz und Dade. Dabei ergibt sich, daß die PTT-Bestimmung durch den Prokallikreintest nicht gestört wird.

## Patentansprüche

1. Verfahren zur simultanen photometrischen Bestimmung von Prokallikrein und der partiellen Thromboplastinzeit (PTT) im Plasma,

dadurch gekennzeichnet, daß man Plasma mit einem Oberflächenaktivator aus der Gruppe Ellagsäure, Sulfatide und feinteiliges Siliciumdioxid inkubiert, dann ein chromogenes Thrombinsubstrat zusetzt, die erhaltene Extinktion mißt und danach die daraus freigesetzte optisch bestimmbare Gruppe in kurzen Zeitabständen oder kontinuierlich mißt und den Anstieg des linearen Teils der beim Auftrag der Zeit gegen die Extinktion erhaltenen Kurve als Maß für den Prokallikreingehalt und die Zeit bis zum Erreichen einer vorbestimmten Extinktion als PTT bestimmt.

2. Verfahren nach Anspruch 1, dadurch gekennzeichnet, daß man der Testlösung auch Ca2+-Ionen zusetzt.

3. Verfahren nach Anspruch 2, dadurch gekennzeichnet, daß man 1 bis 100 µmol pro ml Ca2+-Ionen zusetzt.

4. Verfahren nach Anspruch 4, dadurch gekennzeichnet, daß man 0,08 bis 2,1 µg Ellagsäure pro ml Testlösung zusetzt.

5. Verfahren nach einem der vorhergehenden Ansprüche, dadurch gekennzeichnet, daß man ein Phospholipid zusetzt.

6. Verfahren nach Anspruch 5, dadurch gekennzeichnet, daß man als Phospholipid Kephalin, Sojabohnenphospholipid, Phosphatidylethanolamin oder Phosphatidylcholin zusetzt.

7. Verfahren nach Anspruch 6, dadurch gekennzeichnet, daß man 4 bis 200 µg Kephalin pro ml Testlösung zusetzt.

8. Verfahren nach einem der vorhergehenden Ansprüche, dadurch gekennzeichnet, daß man ein chromogenes Thrombinsubstrat aus der Gruppe Tos-Gly-Pro-Arg-pNA, H-D-CHA-Ala-Arg-pNA, H-D-CHG-Gly-Arg-pNA, H-D-CHG-Ala-Arg-pNA, H-D-CHG-but-Arg-pNA, Iso-Buco-Gly-Arg-pNA, Naphthyl-SO$_2$-Gly-Pro-Arg-pNA, H-D-Phe-Pip-Arg-pNA und Bz-Pro-Phe-Arg-pNA verwendet.

9. Verfahren nach einem der vorhergehenden Ansprüche, dadurch gekennzeichnet, daß man bei 20 bis 40°C, insbesondere 30 bis 37°C inkubiert.

10. Verfahren nach einem der vorhergehenden Ansprüche, dadurch gekennzeichnet, daß man 1 bis 10 Minuten inkubiert.

## Claims

1. Process for the simultaneous photometric determination of prokallikrein and of the partial thromboplastin time (PTT) in plasma, characterised in that one incubates plasma with a surface activator of the group ellagic acid, sulphatides and finely-divided silicon dioxide, then adds thereto a chromogenic thrombin substrate, measures the extinction obtained and thereafter measures the optically determinable group liberated therefrom at short time intervals or continuously and determines the gradient of the linear part of the curve obtained by plotting the time against the extinction as measure of the prokallikrein content and the time up to the achievement of a predetermined extinction as PTT.

2. Process according to claim 1, characterised in that one also adds Ca2+ ions to the test solution.

3. Process according to claim 2, characterised in that one adds 1 to 100 µmol per ml. Ca2+ ions.

4. Process according to claim 1, characterised in that one adds 0.08 to 2.1 µg. of ellagic acid per ml. of test solution.

5. Process according to one of the preceding claims, characterised in that one adds a phospholipid.

6. Process according to claim 5, characterised in that one adds cephalin, soya bean phospholipid, phosphatidylethamine or phosphatidylcholine as phospholipid.

7. Process according to claim 6, characterised in that one adds 4 to 200 µg cephalin per ml. of test solution.

8. Process according to one of the preceding claims, characterised in that one uses a chromogenic thrombin substrate of the group Tos-Gly-pro-Arg-pNA, H-D-CHA-Ala-Arg-pNA, H-D-CHG-Gly-Arg-pNA, H-D-CHG-Ala-Arg-pNA, H-D-CHG-but-Arg-pNA, Iso-Buco-Gly-Arg-pNA, naphthyl-SO$_2$-Gly-Pro-Ard-pNA, H-D-Phe-Pip-Arg-pNA and Bz-Pro-Phe-Arg-pNA.

9. Process according to one of the preceding claims, characterised in that one incubates at 20 to 40°C., especially at 30 to 37°C.

10. Process according to one of the preceding claims, characterised in that one incubates for 1 to 10 minutes.

## Revendications

1. Procédé de détermination photométrique simultanée de la prokallikréine et du temps de thromboplastine partiel (PTT) dans le plasma, caractérisé en ce qu'on fait incuber le plasma avec un agent tensioactif choisi parmi l'acide ellagique, les sulfatides, et un dioxyde de silicium finement divisé, puis en ce au'on ajoute un substrat de la thrombine chromogène, en ce qu'on mesure l'extinction obtenue, puis en ce qu'on mesure le groupe déterminable optiquement libéré de celui-ci à de courts intervalles de temps ou en continu, et en ce qu'on détermine la pente de la partie linéaire de la courbe obtenue en portant le temps en fonction de l'extinction en tant que mesure de la teneur en prokallikréine et le temps jusqu'a obtention d'une extinction déterminée à l'avance en tant que PTT.

2. Procédé suivant la revendication 1, caractérisé en ce qu'on ajoute également des ions Ca2+ à la solution d'essai.

3. Procédé suivant la revendication 2, caractérisé en ce qu'on ajoute 1 à 100 µmoles/ml d'ions Ca2+.

4. Procédé suivant la revendication 1, caractérisé en ce qu'on ajoute 0,08 à 2,3 µg d'acide ellagique par ml de solution d'essai.

5. Procédé suivant l'une des revendications précédentes, caractérisé en ce qu'on ajoute un phospholipide.

6. Procédé suivant la revendication 5, caractérisé en ce qu'on ajoute comme phospholipide de la céphaline, un phospholipide du soja, la phosphatidyléthanolamine ou la phosphatidylcholine.

7. Procédé suivant la revendication 6, caractérisé en ce qu'on ajoute 4 à 200 µg de céphaline par ml de solution d'essai.

8. Procédé suivant l'une des revendications précédentes, caractérisé en ce qu'on utilise un substrat de thrombine chromogène choisi parmi Tos-Gly-Pro-Arg-pNA, H-D-CHA-Ala-Arg-pNA, H-D-CHG-Gly-Arg-pNA, H-D-CHG-Ala-Arg-pNA, H-D-CHG-but-Arg-pNA, Isi-Buco-Gly-Arg-pNA, naphtyl-SO₂-Gly-Pro-Arg-pNA, H-D-Phe-Pip-Arg-pNA et Bz-Pro-Phe-Arg-pNA.

9. Procédé suivant l'une des revendications précédentes, caractérisé en ce qu'on fait incuber à 20 à 40°C, en particulier à 30 à 37°C.

10. Procédé suivant l'une des revendications précédentes, caractérisé en ce qu'on fait incuber 1 à 10 minutes.

# FIG.1

$\Delta E / min$
$\lambda = 405 \ nm$

Prokallikrein in Plasma

1

# FIG.2

# FIG.3